# EUROPEAN PATENT SPECIFICATION

(11) **EP 1 308 173 B1**
(45) Date of publication and mention of the grant of the patent: **24.12.2008**
(21) Application number: 02257623.5
(22) Date of filing: 04.11.2002
(51) Int. Cl.: A61L 2/20, A61L 2/26, A61L 2/18

(54) **Apparatus and process for concentrating a sterilant and sterilizing articles with the concentrated sterilant**
Einrichtung und Verfahren zum Aufkonzentrieren eines Sterilisationsmittels und Sterilisation von Objekten mit dem aufkonzentrierten Sterilisationsmittel
Procédé et dispositif de concentration d'un stérilisant et stérilisation d'objets avec le stérilisant concentré

(30) Priority: 05.11.2001 US 8017
(43) Date of publication of application: 07.05.2003
(73) Proprietor: ETHICON, INC., Somerville, NJ 08876 (US)
(72) Inventor: Williams, Hal, San Clemente, CA 92673 (US); Lin, Szu-Min, Laguna Hills, CA 92653 (US); Fryer, Ben, Lake Forest, CA 92609 (US)
(74) Representative: Fisher, Adrian John

(56) References cited:
- WO-A-00/38745
- WO-A-00/38746
- US-A- 4 744 951

## Description

### FIELD OF THE INVENTION

The present invention relates to a process and an apparatus for sterilization of medical instruments using a chemical sterilant. More particularly, the invention relates to a process and an apparatus in which sterilization is achieved by concentrating a sterilant such as hydrogen peroxide and sterilizing articles therewith.

### BACKGROUND OF THE INVENTION

Medical instruments have traditionally been sterilized using either heat, such as is provided by steam, or a chemical, such as formaldehyde or ethylene oxide in the gas or vapor state. Each of these methods has its drawbacks. Many medical devices such as fiberoptic devices, endoscopes, power tools, etc., are sensitive to heat, moisture or both. Formaldehyde and ethylene oxide are both toxic gases that pose a potential hazard to healthcare workers. Problems with ethylene oxide are particularly severe, because its use requires long aeration times to remove the gas from articles that have been sterilized. This lengthens the sterilization cycle time undesirably.

Sterilization using liquid hydrogen peroxide solution has been found to require high concentrations of sterilant, extended exposure time and/or elevated temperatures. However, sterilization using hydrogen peroxide vapor has been shown to have some advantages over other chemical sterilization processes (see, e.g., U.S. -A- 4,169,123 and 4,169,124.

The combination of hydrogen peroxide with a plasma provides certain additional advantages, as disclosed in U.S. -A- 4,643,876. Commercially available sterilization devices, such as the STERRAD® sterilization systems sold by Advanced Sterilization Systems division of Ethicon, Inc. automate the process of injecting a solution of hydrogen peroxide into a sterilization chamber, vaporizing the solution to provide a hydrogen peroxide vapor, contacting articles to be sterilized with the vapor, and exciting the vapor into the plasma phase. The hydrogen peroxide for each sterilization cycle is shipped to the location of the sterilization system, generally by air or ground transportation.

Preferably, as in the case with the STERRAD^{®} brand systems, pre-measured amounts of a hydrogen peroxide and water solution are provided in sealed enclosure, such as a capsule inside of a cassette housing which can be automatically opened by the system to reduce contact between the system user and the hydrogen peroxide solution. Such cassettes are described more fully in U.S. -A- 4,817,800.

The sterilization of articles containing diffusion-restricted areas, such as long narrow lumens, presents a special challenge. Methods that use hydrogen peroxide vapor that has been generated from an aqueous solution of hydrogen peroxide have certain disadvantages. One disadvantage is that because water has a higher vapor pressure than hydrogen peroxide, it will vaporize faster. Another disadvantage is that because of its lower molecular weight, water will diffuse faster than hydrogen peroxide in the vapor state. Because of these physical properties, when an aqueous solution of hydrogen peroxide is vaporized in the area surrounding the items to be sterilized, the water reaches the items first and in higher concentration. The water vapor more quickly diffuses into and thus inhibits penetration of hydrogen peroxide vapor into diffusion-restricted areas, such as small crevices and long narrow lumens. Simply employing a more concentrated solution of hydrogen peroxide fails to adequately address the problem due to the difficulty in handling highly concentrated hydrogen peroxide solutions. Transportation of such solutions can be particularly difficult. In general, such solutions are limited to concentrations of less than 60% hydrogen peroxide, however, regulations and the like regarding such concentrations may of course be modified in the future. In any event, shipping and handling of highly concentrated solutions remains impractical.

U.S. -A- 4,744,951 attempts to address this problem by providing a separate prechamber connected to the sterilization chamber. Hydrogen peroxide is first admitted to the prechamber where it is concentrated in a distillation procedure employing the differing vapor pressures of hydrogen peroxide and water. Water's higher vapor pressure allows one to select a vaporization pressure that selectively vaporizes water from a hydrogen peroxide solution, thus concentrating the solution. Cummings pumps air out of the prechamber and lowers its pressure to a level at which the water preferentially vaporizes from the hydrogen peroxide solution. The pump that is evacuating the prechamber draws out the water vapor thus released from solution to concentrate the remaining solution. To prevent the water vapor from traveling into the narrow spaces such as endoscope lumens, Cummings carries out the concentration process in the prechamber which is physically isolated from the main chamber. This adds complexity by requiring additional chambers, pumps and valves.

The apparatus disclosed in US-A- 4 744 951 comprises
a source of liquid germicide comprising hydrogen peroxide;
a chamber adapted to receive the article;
a first vaporizer; and
a pump
wherein the first vaporizer and the pump are in fluid communication with the chamber through a common port.

U.S. -A- 4,952,370 discloses a sterilization process in which aqueous hydrogen peroxide vapor is first condensed on the article to be sterilized, followed by application of a vacuum to the sterilization chamber to remove the water and hydrogen peroxide from the article. This method is suitable for surface sterilization, but not for sterilization of diffusion-restricted areas such as long narrow lumens because it depends on the diffusion of hydrogen peroxide vapor into the lumen to effect sterilization.

U.S. -A- 4,943,414 discloses a process in which a vessel containing a small amount of a vaporizable liquid sterilant solution is attached to a lumen, and the sterilant vaporizes and flows directly into the lumen of the article as the pressure is reduced during the sterilization cycle. This system has the advantage that the water and hydrogen peroxide vapor are pulled through the lumen by the existing pressure differential, increasing the sterilization rate for lumens, but has the disadvantage that the vessel needs to be attached to each lumen to be sterilized.

U.S. -A- 5,492,672 discloses a process for sterilizing narrow lumens. This process uses a multi-component sterilant vapor and requires successive alternating periods of flow of sterilant vapor and discontinuance of such flow. A complex apparatus is used to accomplish the method. Because flow through of vapor is used, closed end lumens are not readily sterilized in the process.

Therefore, there is a need to have an easier simpler apparatus and method to concentrate the peroxide solution without the need to isolate the vaporizer from the chamber, and then use the concentrated peroxide to sterilize the articles in the chamber.

A sterilization apparatus and method in accordance with Figures 1 to 9 hereof are disclosed in WO-A-00/38745.

### SUMMARY OF THE INVENTION

According to the present invention, there is provided an apparatus for sterilizing an article, as defined in claim 1. Also provided is a method of sterilizing an article using the apparatus. The method, is defined in claim 8, comprises the steps of: placing the article into a chamber containing an inner atmosphere; introducing a solution comprising hydrogen peroxide and water into a vaporizer in fluid communication with the chamber through a port, the solution having a ratio of hydrogen peroxide to water; pumping a portion of the inner atmosphere out of the chamber through the port thereby reducing pressure in the chamber and in the vaporizer; vaporizing water vapor out of the solution; drawing the water vapor from the vaporizer to increase the ratio of hydrogen peroxide to water in the vaporizer; terminating the pumping step without terminating the vaporizing steps; vaporizing hydrogen peroxide vapor out of the solution and flowing the hydrogen peroxide vapor from the vaporizer into the chamber; and contacting the article with the hydrogen peroxide vapor for a time period sufficient to effect sterilization of the article.

Preferably the ratio of hydrogen peroxide to water in said solution, by weight, after the step of drawing water vapor from the vaporizer exceeds 3 to 1, and more preferably exceeds 4 to 1. The ratio of hydrogen peroxide to water in solution prior to drawing the water is preferably less than 3 to 2.

The step of drawing water vapor from the vaporizer preferably comprises introducing said solution within a diffusion restricted environment in fluid communication with the chamber during the step of vaporizing the solution. The diffusion restricted environment is preferably more diffusion restricted during the step of drawing water vapor from the vaporizer than during a portion of the step of flowing the hydrogen peroxide vapor from the vaporizer into the chamber.

At least a portion of the pumping step and the vaporizing step can occur simultaneously, and at least a portion of the pumping step, the vaporizing step and the drawing step can occur simultaneously.

Preferably, the step of drawing water vapor from the vaporizer comprises the step of maintaining the solution at a pressure below the vapor pressure of the water in the solution and above the vapor pressure of the hydrogen peroxide in the solution. The temperature of the solution during the vaporizing step can be held below the temperature of the atmosphere in the chamber whereby to increase the vapor pressure of the water in the solution relative to the hydrogen peroxide in the solution whereby to enhance vaporization of the water from the solution in preference to vaporizing the hydrogen peroxide from the solution. For instance, the temperature of the atmosphere in the chamber can be above room temperature with the temperature of the solution during the vaporizing step at least 10° C below the temperature of the atmosphere in the chamber. To this end, the vaporizer can be thermally isolated from the chamber.

The solution can be vaporized by pumping a portion of the atmosphere out of the vaporizer to lower the pressure at a rate selected to control removal of the water and hydrogen peroxide from the solution so as to concentrate the hydrogen peroxide remaining in the vaporizer. One can control the temperature and pressure of the solution during a least a first portion of the vaporizing step so as to vaporize water from the solution and concentrate hydrogen peroxide therein to form a concentrated solution and then raise the temperature of and vaporized this concentrated solution.

The step of contacting the article with the hydrogen peroxide vapor can be limited to less than one hour and if the article were to have a straight round lumen having two open ends, a diameter of 1mm and a length of 250mm with 10⁶ viable spores of B. Stearothermophilus located within the lumen at a midpoint thereof, all of the spores would be killed.

Air can be bled into said port from exterior of the chamber, preferably from a location between the vaporizer and the chamber. The step of terminating the pumping step can comprise closing fluid communication between the pump and the port, as with a valve. In a defined flow stream between the chamber and the pump, the vaporizer is preferably located between the chamber and the pump.

An apparatus according to the present invention is adapted for sterilizing an article with a concentrated hydrogen peroxide vapor. The apparatus comprises a source of liquid germicide comprising hydrogen peroxide; a chamber adapted to receive the article, wherein said chamber has a port; a vaporizer in fluid communication with the chamber through the port; and a pump in fluid communication with the chamber through the port.

Preferably, a valve is provided to isolate the pump from the chamber and the vaporizer.

An inlet can be provided to bleed air into the apparatus. It is preferably located either in the chamber or between the vaporizer and the chamber. Preferably, the inlet comprises a valve.

A second vaporizer can be provided in fluid communication with the chamber through the port. In which case a valve can be provided between the first vaporizer and the second vaporizer, to isolate the second vaporizer from the first vaporizer. Also, an inlet can be located between the first vaporizer and the second vaporizer, to bleed air into the apparatus.

### BRIEF DESCRIPTION OF THE DRAWINGS

FIG. 1 is a schematic diagram of a chamber and accessories suitable for use in a known hydrogen peroxide sterilization process.
FIG. 2 is a schematic diagram of a chamber, pump and throttle valve for use in a known hydrogen peroxide sterilization process.
FIG. 3 is a schematic diagram of a system with one pump and two valves, one valve having a larger pump vacuum line for quicker pumpdown and one having a smaller vacuum line for slower pumpdown.
FIG. 4 is a schematic diagram of a single valve sterilization system having two pumps, one for slower pumpdown and one for quicker pumpdown.
FIG. 5 is a schematic diagram of a system with two pumps and two valves, one pump for slower pumpdown and one for quicker pumpdown.
FIG. 6 is a schematic diagram of a system with a vaporizer.
FIG. 7 is a schematic diagram of a system with an alternative vaporizer.
FIG. 8 is a schematic diagram of a system with a further alternative vaporizer.
FIG. 9 is a graph showing the pressure and vapor peroxide concentration during a concentrating process.
FIG. 10 is a schematic diagram of a system with a vaporizer and a pump connected to the sterilizer through the same port, in accordance with the present invention.
FIG. 11 is a schematic diagram of a system with more than one vaporizer and a pump connected to the sterilizer through the same port, in accordance with the present invention.

### DETAILED DESCRIPTION OF THE PREFERRED EMBODIMENTS

Sterilizing the inside of lumened devices has always posed a challenge to sterilization systems. U.S. -A- 6030579, and its related issued U.S. -A- 5,980,825, disclose a method of hydrogen peroxide vapor sterilization of diffusion-restricted environments, such as long narrow lumens, at pressures less than the vapor pressure of hydrogen peroxide by pretreating the article to be sterilized with a dilute solution of hydrogen peroxide prior to exposure to a vacuum. U.S. Patent 5,851,485 controls the pumpdown rate.

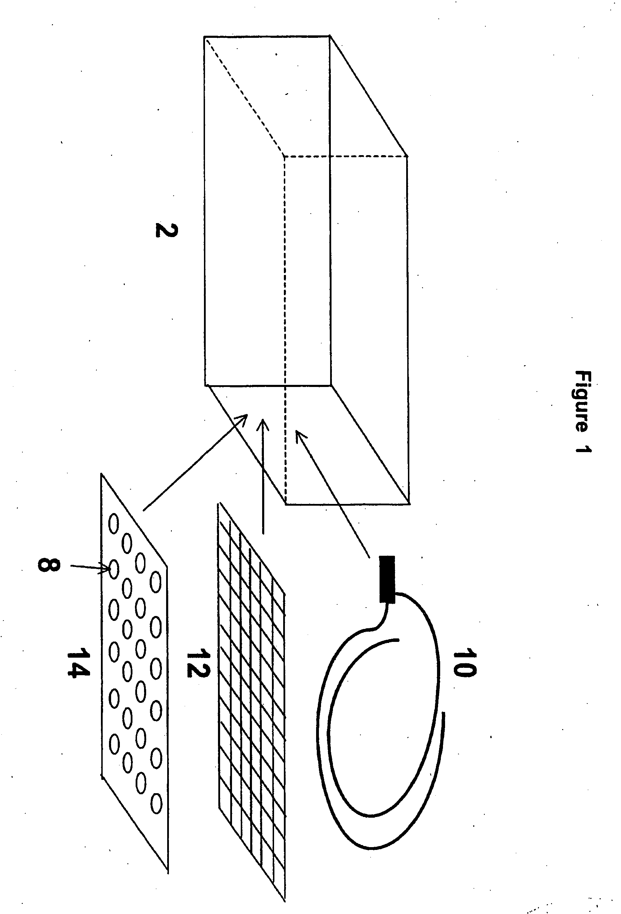

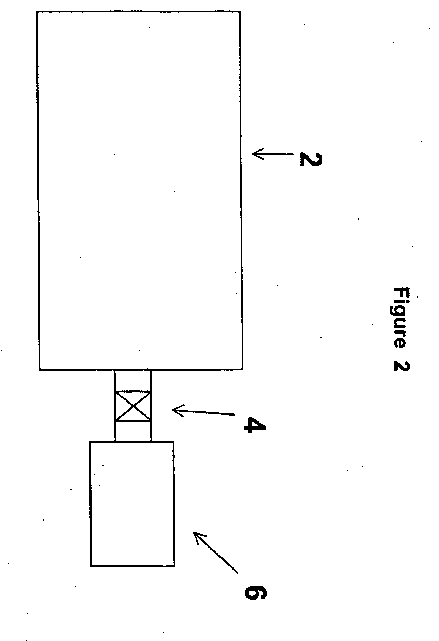

An apparatus as disclosed in WO-A-00/38745 is shown schematically in FIGS. 1 and 2 and comprises a chamber 2, a throttle valve 4 and a pump 6. In FIG. 2, the chamber 2 is attached to the pump 6 by the throttle valve 4. The valve 4 can be controlled either automatically or manually to control the pressure. In the automatic mode of operation, the throttle valve 4 opens based on the pressure in the chamber via a pressure transducer and valve controller. Such valves are commercially available from, for example, MKS (Andover, MD).

According to WO-A-00/38745, hydrogen peroxide can be introduced into the system in any fashion. In one embodiment, a dilute, aqueous solution of hydrogen peroxide is placed in wells 8 as shown in FIG. 1. The aqueous solution of hydrogen peroxide can also be placed within the lumen of long narrow objects to be sterilized. As the pressure in the sterilization chamber 2 is reduced, the hydrogen peroxide vaporizes and contacts the surface to be sterilized (i.e., colonoscope 10 in FIG. 1) which is placed on metal grid 12 which rests on tray 14. In one embodiment, the tray can be configured with a plurality of wells designed to retain a known volume of liquid sterilant. In one embodiment, the volume of sterilization chamber 2 is about 18.5 liters and its dimensions are about 22" (55.9 cm) x 4.25" (10.8 cm) x 12" (30.5 cm).

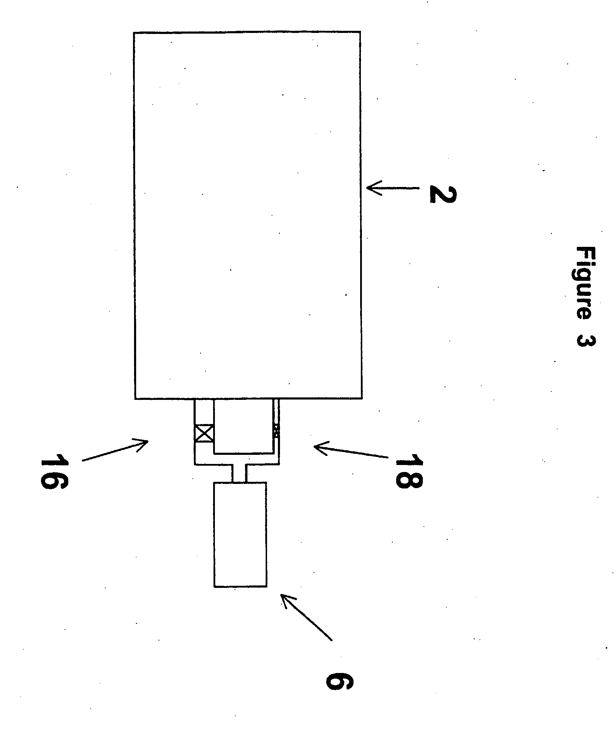

FIG. 3 illustrates a parallel two-valve arrangement for use in the known sterilization process. In this embodiment, the chamber 2 is in fluid communication with the pump 6 via valves 16 and 18. Valve 16 mediates the initial rapid evacuation, the first step of a two step evacuation process. Valve 18 mediates slow evacuation, the second step of the process, which ensures maximal contact of the article to be sterilized with the vaporized aqueous hydrogen peroxide. The pumpdown rate can be controlled by the pumping speed and/or the percent opening of the valve. Either valve can be used to control the pressure. In practice, controlling the process so that nearly all of the water evaporates before any of the hydrogen peroxide evaporates is very difficult, yet the preferential evaporation and elimination of water vapor from the system effectively concentrates the hydrogen peroxide therein without the attendant complexity of shipping and handling concentrated hydrogen peroxide solutions prior to vaporization.

As the water evaporates from the solution, the number of its molecules in the vapor state greatly increases thus raising the pressure in the system and requiring additional pumping to extract the water vapor and other molecules to control the pressure. Also, the vapor pressures change with changing conditions within the chamber.

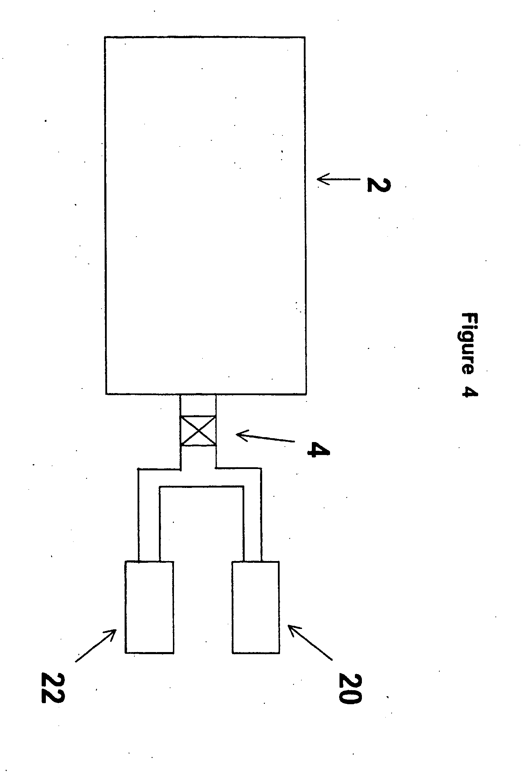

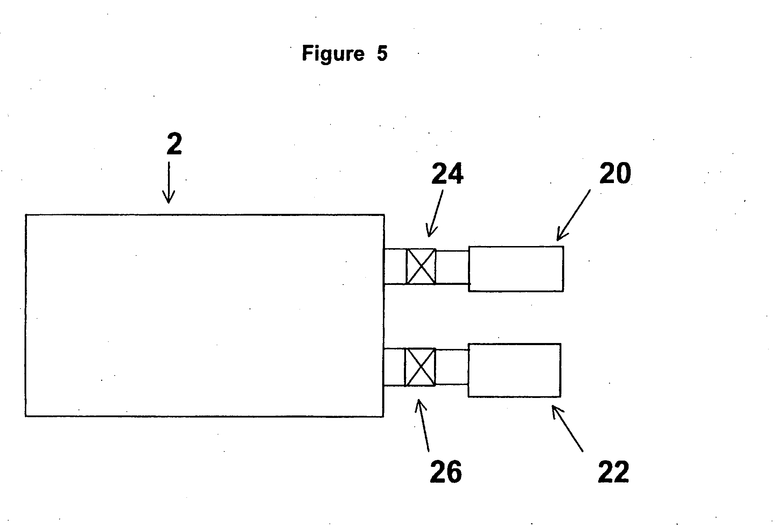

FIG. 4 illustrates a sterilization apparatus having two pumps 20 and 22, and one valve 4. Pump 20 allows quicker pumpdown of the chamber 2, while pump 22 allows slower pumpdown. FIG. 5 illustrates an alternate configuration having two valves 24 and 26 in fluid communication with the pumps 20 and 22, respectively.

According to WO-A-00/38745, hydrogen peroxide can be introduced into the chamber as a liquid. In one preferred embodiment, hydrogen peroxide is introduced as a vapor and the chamber parameters are changed so that the vapor condenses as a liquid on the surface of interior of an article to be sterilized. Such changes include increasing the partial pressure of hydrogen peroxide and/or decreasing the temperature.

The aqueous solutions of hydrogen peroxide can be relatively dilute, e.g. as low as 1-6% peroxide by weight, since sterilization is not achieved through contact with the hydrogen peroxide solution, but rather is achieved at low temperatures (preferably 15°-80°C, more preferably 20°-60°C, still more preferably 40°-55°C) and in short periods of time (preferably less than one hour, and more preferably less than one-half hour) upon exposure to hydrogen peroxide under vacuum. The method is particularly effective with articles having diffusion-restricted areas. Such articles include long, narrow lumens, hinges and other articles having spaces where diffusion of vapors is restricted.

Preferably, the article to be sterilized is contacted with liquid sterilant prior to the vaporization step to localize at least some of the vaporization in the diffusion-restricted areas. Such contacting can be accomplished either directly or indirectly. Direct contacting includes methods such as static soaking, flow through, or aerosol spray, or condensation of a vapor. Any other methods involving physically contacting the articles to be sterilized with sterilant would be considered direct contacting. Indirect contacting includes those methods in which sterilant is introduced into the chamber, but not directly on or in the articles to be sterilized.

At the end of the process, deep vacuum can be used to remove residual sterilant. A plasma can also be used to both enhance sterilization efficacy and to remove residual sterilant.

The pumps shown schematically in the figures can be any commercially available vacuum pump. Two preferred pumps are from Leybold Vacuum Products, Inc. (Export, PA) (Model D16A, pump rate = 400 liters/min) and KNF Neuberger, Inc. (Trenton, NJ, Model N740, pump rate = 45 liters/min). The Leybold pump can reach a pressure of less than 0.1 torr and the KNF pump can reach a pressure of less than 10 torr.

For certain substrates being sterilized, such as nylon or polyurethane, excess hydrogen peroxide in the system may leave a residual which is difficult to be removed. In order to avoid an excess residual, the vapor concentration of hydrogen peroxide is preferably kept below 30 mg/l, more preferably less than 20 mg/l, and more preferably still less than 15 mg/l. If higher vapor concentrations of hydrogen peroxide are desired, excess residual can be removed using gas plasma. When using substrates such as stainless steel, polyethylene or polypropylene, which do not retain a residual, substantially more peroxide can be present in the vapor phase in the system during sterilization.

To further reduce water within the system, the chamber 2 and the load within the chamber may be dried prior to the introduction of hydrogen peroxide. Many means may be employed to drive water out of the chamber. Primarily, this is accomplished by vaporizing the water and pumping it out of the chamber. The vaporization can be accomplished with heat, plasma induction, vacuum or the like, either alone or in combination. Merely drawing a vacuum prior to introducing the hydrogen peroxide accomplishes a beneficial drying of the chamber 2. If the chamber 2 is heated during this process and if a high energy electromagnetic field is applied to urge the water into the plasma phase the drying is enhanced. U.S. Patent No. 5,656,238 issued on August 12, 1997 to Spencer at al. and incorporated herein by reference teaches such techniques in more detail.

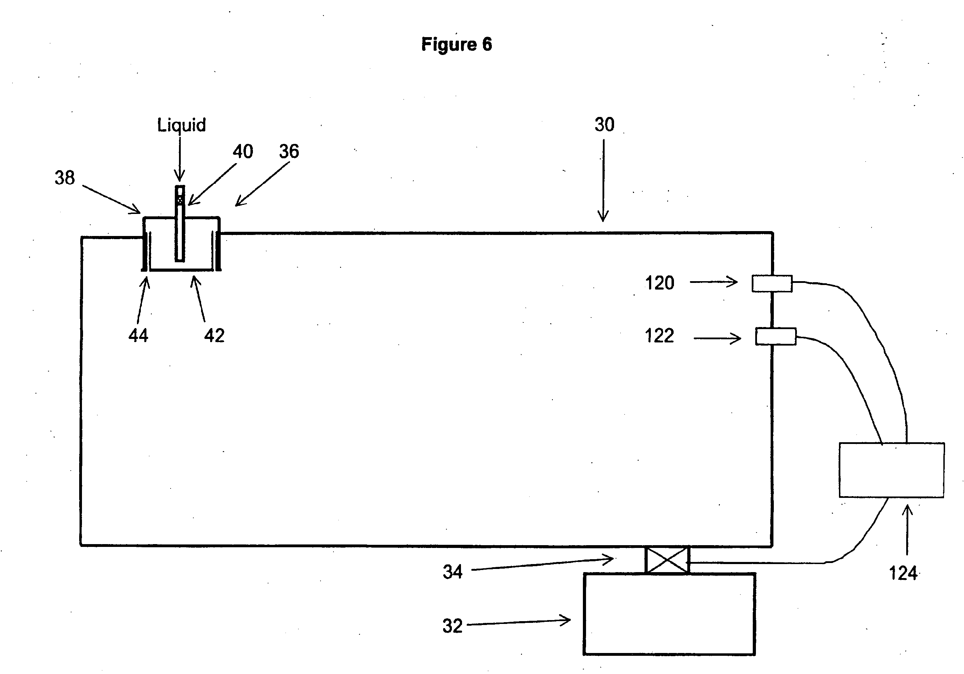

Vaporization of the hydrogen peroxide can be achieved using well known methods as described above; FIGS. 6 to 8 show several preferred methods. In FIG. 6, a chamber 30 is evacuated by a pump 32 separated from the chamber 30 by a throttle valve 34. A vaporizer 36 comprises a housing 38 in fluid communication with the chamber 30 and into which extends a liquid feeding nozzle 40 from outside of the chamber 30. A cup 42 within the housing 38 receives hydrogen peroxide from the nozzle 40. The hydrogen peroxide can be vaporized as it exits the nozzle 40, or more preferably in a controlled fashion from the cup 42 by controlling the temperature of the cup 42 and the pressure in the chamber 30. Temperature control of the cup 42 can be as simple as thermally isolating it from the chamber 30, or a more active control system can be employed such a cooling coil or the like to maintain the cup 42 at a desired low temperature. Preferably, the entire vaporizer 36 is thermally isolated from the chamber 30 or temperature controlled in some fashion. Lower temperatures of vaporization enhance the preferential vaporization of water by exploiting the larger difference between the vapor pressures of water and hydrogen peroxide at lower temperatures. Creating a diffusion restriction 44 between the vaporizer 36 and chamber 30 enhances the preferential extraction of water vapor from the chamber as water vapor will more easily traverse the diffusion restriction and be pumped out of the chamber during the vaporization process. The diffusion restriction 44 may be simply reducing the clearance between the cup 42 and housing 38 through with the vapor must travel to reach the chamber 30.

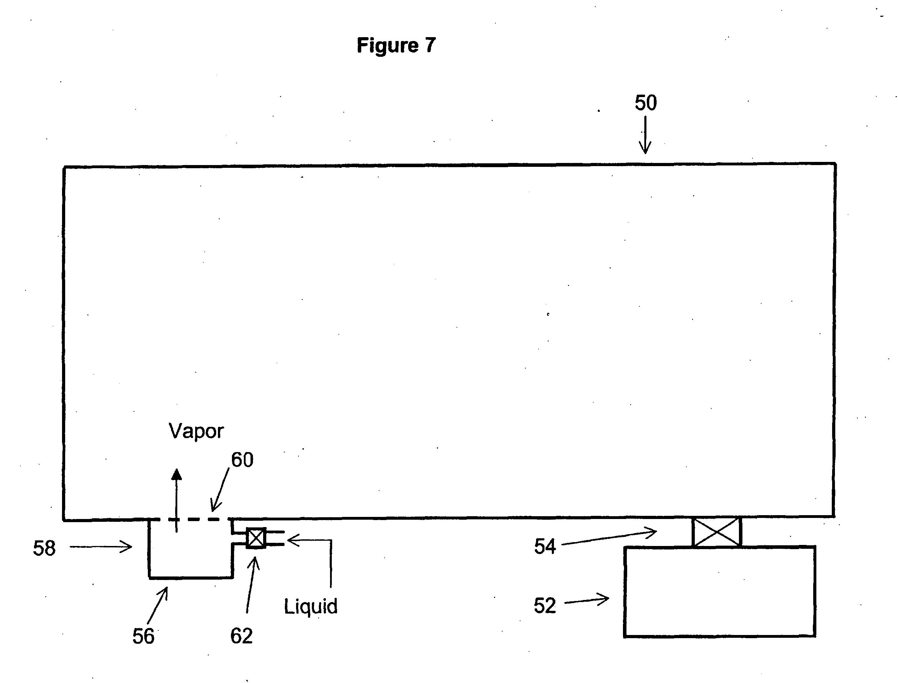

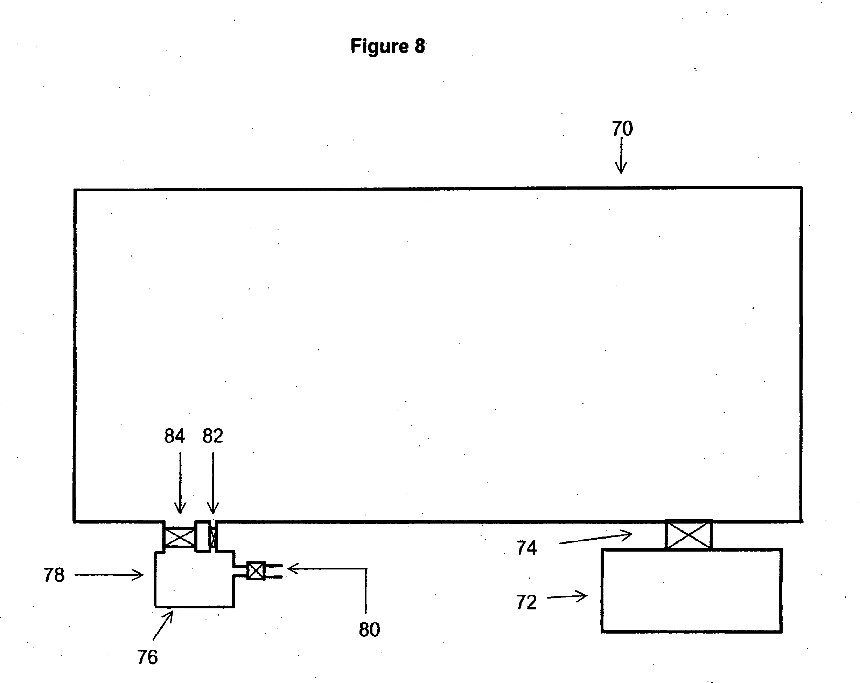

FIG. 7 shows a similar chamber 50, pump 52 and valve 54 with modified vaporizer 56. The vaporizer 56 comprises a chamber 58 separated from the chamber 50 by a diffusion restriction 60, such as a permeable membrane. Liquid hydrogen peroxide solution enters the chamber 58 through a valve 62. FIG. 8 illustrates a similar arrangement with a chamber 70, pump 72, valve 74, and vaporizer 76 with a chamber 78 and valved hydrogen peroxide solution inlet 80. Restriction of the diffusion between the vaporizer chamber 78 and main chamber 70 is variable. During initial vaporization when primarily water is vaporizing the vapors pass through a tight diffusion restriction 82. After the concentration of the hydrogen peroxide solution reaches a given level valve 84 may be opened to speed the vaporization and diffusion of the concentrated hydrogen peroxide solution.

Preferably, the temperature in the chamber is no less than 5°C nor more than 150° C, with the range of 40 to 60°C being preferred, and the pressure should be no less than 0.01 torr, nor typically greater than atmosphere during the process, with the lowest vacuum being typically 0.1 torr and the diffusion pressure preferably being between 1 and 15 torr, although other conditions within the spirit of the invention will be apparent to those of skill in the art. Preferably, during the concentration phase, the vaporizer pressure does not fall below 0.3 torr. Shorter overall cycles are preferred for convenience, with 5 minutes being a desirable goal, but longer times upwards of 6 hours or more may be warranted in some circumstances.

Tables 1 and 2 illustrate the effectiveness of the concentrating process with the apparatus as shown in FIG 6. The experiments were run on a chamber of 73 liters at 45°C with 1480 mg of 59% hydrogen peroxide solution by weight. The vaporizer is separated from the chamber by twelve 2 mm diameter holes to effect diffusion restriction. Test A was conducted by opening the valve, evacuating the chamber to 0.3 torr, closing the valve, injecting the peroxide solution into the vaporizer, allowing the water and peroxide to vaporize and diffuse, and venting the chamber. Test B was conducted by injecting peroxide solution into the vaporizer at the atmospheric pressure, opening the valve, evacuating the chamber to 2 torr, closing the valve, allowing the remaining water and peroxide to vaporize and diffuse, and venting the chamber. Test C was conducted by opening the valve, evacuating the chamber, injecting the peroxide solution into the vaporizer when the chamber was evacuated to 30 torr, continuing to evacuate the chamber to 2 torr, closing the valve, allowing the remaining water and peroxide to vaporize and diffuse, and venting the chamber. The procedure for test D was same as test C except the peroxide solution was introduced into the vaporizer at 0.3 torr. Test E was conducted by opening the valve, evacuating the chamber to 0.3 torr, closing the valve, injecting the peroxide solution into the vaporizer, allowing the water and peroxide to vaporize and diffuse for 30 seconds, opening the valve, evacuating the chamber to 2 torr, closing the valve, allowing the remaining water and peroxide to vaporize and diffuse, and venting the chamber.

**Table 1**

| Step | Test conditions | | | | |
|---|---|---|---|---|---|
| | Normal process | New concentrating Process | | | |
| | Test A | Test B | Test C | Test D | Test E |
| 1 | Open valve | Inject H₂O₂ at 1 atm | Open valve | Open valve | Open valve |
| 2 | Vacuum to 0.3 torr | Open valve | Vacuum to 30 torr | Vacuum to 0.3 torr | Vacuum to 0.3 torr |
| 3 | Close valve | Vaporization & diffusion | Inject H₂O₂ at 30 torr | Inject H₂O₂ at 0.3 torr | Close valve |
| 4 | Inject H₂O₂ at 0.3 torr | Vacuum to About 2 torr | Vaporization & diffusion. | Vaporization & diffusion | Inject H₂O₂ at 0.3 torr |
| 5 | Vaporization & diffusion | Close valve | Vacuum to About 2 torr | Vacuum to About 2 torr | Vaporization & diffusion |
| 6 | Vent to 1 atm | Vaporization & diffusion | Close valve | Close valve | Open valve |
| 7 | | Vent to 1 atm | Vaporization & diffusion | Vaporization & diffusion | Vacuum to About 2 torr |
| 8 | | | Vent to I atm | Vent to 1 atm | Close valve |
| 9 | | | | | Vaporization & diffusion |
| 10 | | | | | Vent to 1 atm |

Table 2 shows the efficacy results with and without the concentrating process in the chamber. The tests were conducted by placing one stainless steel wire inoculated with 4.3x10⁶ Bacillus stearothermophilus spores at the center of the stainless steel lumen. Four 1 mm lumens with length ranging from 250 mm to 400 mm were used for each test. All the experiments were conducted by controlling the time between the injecting of peroxide solution and the venting of the chamber to 6 minutes. The results indicate that the new concentrating process is more efficacious than the normal process which does not concentrate the peroxide in the chamber. These results also indicate that the peroxide solution can be introduced before evacuating the chamber, during evacuating the chamber with pressure above or below the vapor pressure of peroxide, or after evacuating the chamber with the valve at the either open or closed position.

**Table 2**

| | Sterility test result (positives / samples) | | | | |
|---|---|---|---|---|---|
| | Normal process | New concentrating Process | | | |
| | Test A | Test B | Test C | Test D | Test E |
| 1 x 400 mm | 2/2 | 0/2 | 0/2 | 0/2 | 0/2 |
| 1 x 350 mm | 2/2 | 0/2 | 0/2 | 0/2 | 0/2 |
| 1 x 300 mm | 2/2 | 0/2 | 0/2 | 0/2 | 0/2 |
| 1 x 250 mm | 2/2 | 0/2 | 0/2 | 0/2 | 0/2 |

Monitoring of the temperature, pressure and hydrogen peroxide conditions within the chamber 30 (FIG. 6) allows the process to be controlled more precisely. Preferably, an automated control system, preferably employing a computer processor, receives signals of the temperature, pressure and perhaps also the hydrogen peroxide concentration and calculates the optimal pressure at which to maintain the chamber to remove the water from the hydrogen peroxide solution and from the chamber 30. It can also determine when the solution is sufficiently concentrated. For instance, it may be desired to only concentrate the solution to a certain degree so as to minimize the loss of hydrogen peroxide from the chamber, thereby minimizing hydrogen peroxide emissions from the chamber. While preferentially vaporizing the water from the solution, some hydrogen peroxide will also vaporize. Accordingly, one may wish to balance the efficient use of the quantity of hydrogen peroxide within the solution against the goal of eliminating all water from the solution and the chamber. By monitoring the ratio of water to peroxide in the vapor phase, the valve 34 can be controlled to remove the vapor until the desired ratio is achieved. The ratio can be determined using a hydrogen peroxide monitor and a moisture monitor, or by using a hydrogen peroxide monitor and a pressure sensor and then calculating the water using the PV=nRT equation and making the assumption that water and peroxide are essentially the only gases within the chamber 30.

It is known that certain spectra of light passing through the chamber can be measured to determine the hydrogen peroxide concentration. One particular method is disclosed in co-pending U.S. -A- 6269680.

Table 3 compares a sterilization process in which the concentration of hydrogen peroxide is not increased with a process in which it is increased. The concentrations of water and peroxide for the normal process without concentrating the peroxide were calculated based on 1480 mg of 59% peroxide solution by weight in a 73 liters chamber. Test E procedure described in Table 1 with the same amount of peroxide solution was used to determine the concentrations of water and peroxide in the chamber with the concentrating process. The concentration of peroxide was measured with a peroxide monitor and the concentration of water was calculated from the pressure and peroxide monitor readings. Unlike the normal process which retains all the peroxide in the chamber, the concentrating process has less available peroxide in the chamber, but it removes more water than peroxide from the chamber and results in more concentrated peroxide for achieving better efficacy.

**Table 3**

| | Normal process | New concentrating process |
|---|---|---|
| Concentration of water | 8.3 mg/L | 1.5 mg/L |
| Concentration of peroxide | 12.0 mg/L | 7.3 mg/L |
| Ratio of peroxide to water by wt | **1.45** | **4.87** |

Table 4 also illustrates effects of the ratio of hydrogen peroxide vapor to water vapor in the chamber 30 on the ability to sterilize long narrow lumens or other diffusion restricted environments with Bacillus subtilis var. niger spores on stainless steel blades in 3mm x 500 mm stainless steel lumen. Water vapor was first introduced into the system and then essentially pure hydrogen peroxide vapor was introduced by liberation from a solid form. The lower concentrations of water show no failures, whereas with the higher ratio in the last column the efficacy decreased and in one test 3 out of 3 samples failed. Therefore, it is desirable to control the amount of water and peroxide in the chamber to achieve better efficacy.

**Table 4**

| | Sterility results (positives / samples) | | |
|---|---|---|---|
| Diffusion time of peroxide (minutes) | 0.653 mg/L water + 6 mg/L peroxide | 3.266 mg/L water + 6 mg/L peroxide | 6.532 mg/L water + 6 mg/L peroxide |
| 5 | 0/3 | 0/3 | 3/3 |
| 10 | 0/3 | 0/3 | 2/3 |
| 15 | 0/3 | 0/3 | 0/3 |
| 30 | 0/3 | 0/3 | 0/3 |

Water vaporizes and diffuses faster than the peroxide under the same temperature and pressure conditions. At the beginning of the injection phase, the ratio of peroxide to water vaporized into the vapor phase is much lower than the ratio of peroxide to water in the liquid introduced into the vaporizer. By leaving the valve at the open position during the injection phase, more water can be removed from the chamber than peroxide. As more water vaporized from the vaporizer and removed from the chamber, the peroxide concentration left in the system is increased. Table 5 shows the degree of concentration achieved according to the present invention by changing the pressure that the valve was closed during the concentrating process with the test E procedure described in Table 1. A total of 1480 mg of 59% by weight hydrogen peroxide solution was used for each test. The results indicate that water is removed faster than the peroxide from the system and the wt% of peroxide is increased by evacuating the system to a lower pressure.

**Table 5**

| | Normal process | New concentrating process | | |
|---|---|---|---|---|
| | | Valve closed at 4 torr | Valve closed at 3 torr | Valve closed at 2 torr |
| Concentration of water | 8.3 mg/L | 3.5 mg/L (58% removed) | 2.7 mg/L (67% removed) | 1.5 mg/L (82% removed) |
| Concentration of peroxide | 12.0 mg/L | 10.5 mg/L (12% removed) | 10.0 mg/L (17% removed) | 7.3 mg/L (39% removed) |
| Ratio of peroxide to water | 1.45 : 1 (59% by wt) | 3.0: 1 (75% by wt) | 3.7 : 1 (79% by wt) | 4.9 : 1 (83% by wt) |

Table 6 discloses another approach to control this concentrating process by directly monitoring the ratio of peroxide to water in the chamber. The valve is then closed when the desired ratio of peroxide to water is reached.

**Table 6**

| | Ratio of peroxide to water | | |
|---|---|---|---|
| | Beginning of the injection phase | Right after concentrating | After all peroxide vaporized |
| Solution in the vaporizer | 1.45 : 1 | 19 : 1 | No solution left in the vaporizer |
| Vapor in the chamber | Very low | 0.85 :1 | 4.9 : 1 |

The test conditions described in Table 5 were repeated with 1780 mg of 59% peroxide solution by weight. Efficacy tests were also conducted under the same conditions with stainless steel wire inoculated with 4.3x10⁶ Bacillus stearothermophilus spores located at the center of the stainless steel lumen. The results, presented in Table 7, clearly indicate that the new concentrating process is more efficacious than the normal process to sterilize lumen devices and all lumens tested with the new concentrating process at three pressure levels were sterilized.

**Table 7**

| | Normal process | New concentrating process | | |
|---|---|---|---|---|
| | | Valve closed at 4 torr | Valve closed at 3 torr | Valve closed at 2 torr |
| Concentration of peroxide | 14.4 mg/L | 6.41 mg/L | 4.52 mg/L | 3.17 mg/L |
| Efficacy with 1 mm x 400mm SS lumen | 2/2 | 0/2 | 0/2 | 0/2 |
| Efficacy with 1mm x 350mm SS lumen | 2/2 | 0/2 | 0/2 | 0/2 |
| Efficacy with 1 mm x 300mm SS lumen | 2/2 | 0/2 | 0/2 | 0/2 |
| Efficacy with 1mm x 250mm SS lumen | 2/2 | 0/2 | 0/2 | 0/2 |

Table 8 shows the efficacy of the concentrating process with 12% peroxide solution by weight. Tests were conducted by placing one stainless steel wire inoculated with 2.1x10⁶ Bacillus stearothermophilus spores at the center of the stainless steel lumen. Four 1 mm lumens with length ranging from 250 mm to 400 mm were used for each test. The normal process was conducted by evacuating the chamber to 0.3 torr, closing the valve, injecting 7400 mg of 12% peroxide solution by weight into the vaporizer, allowing the water and peroxide to vaporize and diffuse for a total of 23 minutes, and venting the chamber. The concentrating process was conducted by evacuating the chamber to 0.3 torr, introducing 7400 mg of 12% peroxide solution by weight into the vaporizer with the valve at the open position, allowing the water and peroxide to vaporize and diffuse, closing the valve when the peroxide concentration increased to 0.45 mg/L, allowing the remaining water and peroxide to vaporize and diffuse, and venting the chamber. Due to the excess of the solution introduced into the vaporizer, the valve was remained at the open position for 16 minutes to remove enough water from the sterilizer and to concentrate the peroxide that remained in the system. The valve was then closed for an additional 7 minutes to allow the remaining peroxide to vaporize and diffuse. The total peroxide exposure time for both processes was 23 minutes. The results, as shown in the Table 8, indicate that the concentrating process is more efficacious than the normal process and diluted peroxide solution can also be used in this concentrating process. These results also indicate that monitoring the peroxide concentration in the chamber can control the concentrating process.

**Table 8**

| | Sterility results (positives / samples) | |
|---|---|---|
| | Normal process | New concentrating Process |
| 1 x 400 mm | 2/2 | 0/2 |
| 1 x 350 mm | 2/2 | 0/2 |
| 1 x 300mm | 2/2 | 0/2 |
| 1 x 250mm | 2/2 | 0/2 |

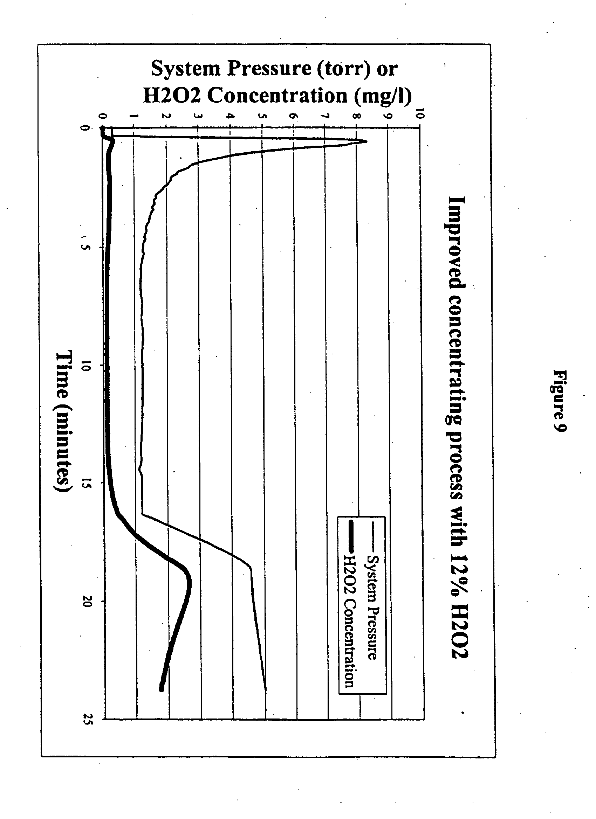

The pressure and peroxide concentration curves during the concentrating process with 12% peroxide solution by weight are presented in Figure 9. The chamber was set at 45°C. The vaporizer has its own heater and is in communication with the chamber and separated from the chamber with the o-rings. Initially, the heater on the vaporizer was off and the vaporizer was heated to about 45°C due to the heated chamber and air around the vaporizer. As indicated from the pressure and peroxide concentration curves, the majority of molecules vaporized and diffused into the chamber during the first 15 minutes were water. Not much peroxide was vaporized and diffused into the chamber. This is consistent with the data published by Schumb et al., as shown in Table 9, that the concentration of hydrogen peroxide in the vapor phase over a 12% peroxide solution by weight, or 6.7% by mole, is less than 0.5% by mole under our test conditions.

As water and peroxide vaporized from the vaporizer, the vaporizer temperature decreased more than 10°C. With the valve at the open position while water and peroxide vaporized and diffused into the chamber, more water is removed from the system than the peroxide, and the peroxide concentration left in the vaporizer is increased. As indicated from the graph, the hydrogen peroxide concentration started to increase after 15 minutes. This indicated that the peroxide solution left in the vaporizer had been concentrated by removing enough water from the vaporizer. The valve was then closed to retain the remaining peroxide vaporized into the sterilizer. The temperature of the vaporizer can then be optionally increased to enhance the vaporization of the remaining peroxide solution left in the vaporizer.

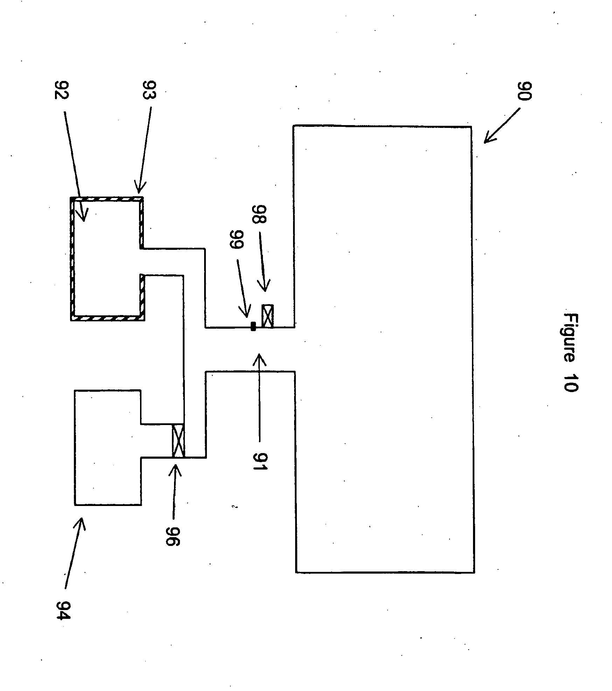

In contrast to the process disclosed in WO-A-00/38745, the concentrating process in accordance with the present invention is conducted by connecting the vaporizer and a vacuum pump to the chamber through a common port. As shown in FIG. 10, both a vaporizer 92 and a pump 94 are in fluid communication with a chamber 90 through a common port 91. This configuration provides the advantage over FIG. 6 of removing the water vapor during the concentrating process directly to the pump 94 without passing it through the chamber 90. By eliminating or reducing the amount of water flow into the chamber, the efficacy of peroxide can be enhanced. The extraction of air from the chamber 90 during the vaporizing process prevents the released vapors from entering the chamber and thereby obviates the need for a valve or other barrier between the vaporizer 92 and the chamber 90.

An optional temperature controller 93 controls the rate of vaporization and an optional valve 96 separates the pump 94 from the vaporizer 92 and chamber 90. Evacuation of the chamber 90 and the vaporizer 92 can be controlled by operation of the valve 96 or by turning the pump 94 on or off.

Another optional valve 98 on the port 91 can flow or bleed air into the system to create a flow from port 91 to the pump 94. This flow hinders flow of water and peroxide from the vaporizer 92 into the chamber 90 during the concentrating process. Alternatively, an optional pinhole 99 can be used to bleed air or gas into the system for the same purpose. The valve 98 or pinhole 99 can also be located on the chamber 90.

After sufficiently concentrating the hydrogen peroxide solution in the vaporizer the pump 94 is turned off or isolated from the chamber by closing the valve 96. The low pressure in the vaporizer vaporizes the remaining concentrated peroxide solution which then diffuses into the chamber 90 through port 91. Methods to perform the concentrating process described in Table 1 can be applied to this apparatus to concentrate the peroxide solution. Various vaporizers, such as the vaporizer 56 of FIG. 7 or the vaporizer 76 of FIG. 8, can also be used with this apparatus to enhance the efficiency of the concentrating process. Preferably, the sterilizer comprises valves 96 and 98. The process parameters, temperature and pressure monitoring and controlling apparatuses and methods previously described can also be applied to this configuration. The drying process may further comprise the use of gas plasma or other heat.

In one of the many operating embodiments, the liquid hydrogen peroxide solution is first introduced into the vaporizer 92. The liquid can be introduced with a cassette delivery system, a bulk delivery system, a syringe, or disposable cell packs or other methods appreciated by those of skill in the art. The pump is then turned on to evacuate the system. This creates two directional flows. The air in the chamber 90 flows from the chamber 90 to the pump 94, and water vapor and peroxide vapor flow from the vaporizer 92 to the pump 94. The flow of air from the chamber 90 to the pump 94 prevents or minimizes the flow of water vapor and peroxide vapor from the vaporizer 92 to the chamber 90. Since water has higher vapor pressure than the hydrogen peroxide, more water is removed from the solution than the hydrogen peroxide. The concentrating efficiency of the hydrogen peroxide solution in the vaporizer 92 can be controlled by controlling the pumping rate, the pressure, and/or the vaporizer temperature. Optionally, the valve 98 and/or a pinhole 99 can be used to further reduce or eliminate the flow of water vapor and peroxide vapor from the vaporizer 92 into the chamber 90. After the peroxide solution is concentrated to a desired concentration, the pump 94 is then turned off or the valve 96 is then closed to allow vaporization of the remaining concentrated peroxide and water from the vaporizer 92 through the port 91 and into the chamber 90. A temperature controller 93 located outside of the vaporizer 92 can be set at various temperatures throughout the cycle to optimize the concentrating process and the vaporization of the concentrated peroxide.

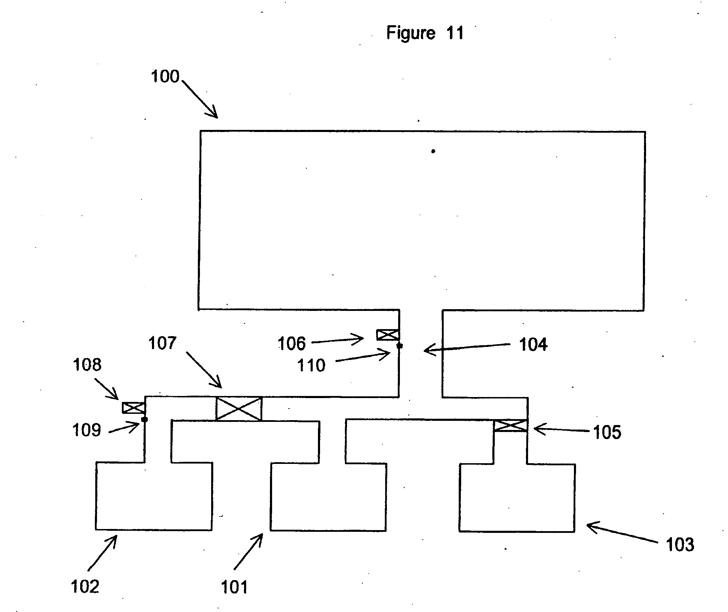

FIG. 11 illustrates an apparatus as in FIG. 10, but with two vaporizers 101 and 102 in fluid communication with a chamber 100 through the port 104. The second vaporizer provides additional dose of peroxide to the chamber. The peroxide solution or the source of peroxide solution can be introduced into the vaporizers at the same time or separately as needed. The vaporizer temperatures can be set differently to independently control the vaporization of the water and peroxide from each vaporizer. Optionally, a valve 105 can be used to isolate the pump 103. Optional valves 106 and 108 and pinholes 109 and 110 can be used to control the direction of the flow of water vapor and peroxide vapor during the concentrating process and/or during the vaporization of the concentrated peroxide solution from the vaporizers 101 and 102 into the chamber 100. Optionally, a valve 107 can be used to separate the vaporizer 102 from the vaporizer 101. Depending on the need, a sterilizer can be designed to have more than two vaporizers.

The length of the concentrating process or the time to close the valve can control the final peroxide concentration or the ratio of the peroxide to the water in the chamber. Since water has a higher vapor pressure than peroxide at the same temperature, the concentration of peroxide in the vaporizer or the chamber can be increased by increasing the time of the concentrating process or by delaying the time to close the valve. This concentrating process can be conducted with peroxide solution in the chamber and/or in the vaporizer which is in fluid communication with the chamber, and it can be enhanced if the environment, which contains the peroxide solution, is a diffusion-restricted area. Monitoring or determining the concentration of water and/or peroxide in the chamber and/or vaporizer can properly control this concentrating process. It is well known in the prior art that the concentration of peroxide is an important factor to achieve good efficacy for the vapor phase peroxide process. Based on the test results of this invention, it is believed that the ratio of peroxide to water is a critical factor in the sterilization process. By determining the concentrations of peroxide and water in the process and calculating the ratio of peroxide to water, parametric release can be achieved. By determining the vapor composition and monitoring the temperature of the peroxide solution, the concentration of the peroxide solution can be determined.

**Table 9**

| Vapor composition (mole fraction H2O2) over hydrogen peroxide water solutions | | | | | | | | | |
|---|---|---|---|---|---|---|---|---|---|
| Temp. (°C) | Mole Fraction Hydrogen Peroxide in Liquid | | | | | | | | |
| | 10% | 20% | 30% | 40% | 50% | 60% | 70% | 80% | 90% |
| 0 | 0.2% | 0.6% | 1.5% | 3.1% | 6.0% | 11.2% | 20.2% | 35.2% | 60.0% |
| 10 | 0.3% | 0.8% | 1.8% | 3.7% | 7.0% | 12.8% | 22.4% | 38.1% | 62.6% |
| 20 | 0.3% | 0.9% | 2.0% | 4.1% | 7.7% | 13.8% | 23.8% | 39.7% | 64.0% |
| 25 | 0.3% | 1.0% | 2.2% | 4.4% | 8.1% | 14.4% | 24.7% | 40.7% | 64.8% |
| 30 | 0.3% | 1.0% | 2.3% | 4.6% | 8.5% | 15.1% | 25.5% | 41.7% | 65.6% |
| 40 | 0.4% | 1.2% | 2.6% | 5.2% | 9.4% | 16.3% | 27.2% | 43.5% | 67.1% |
| 50 | 0.5% | 1.4% | 3.0% | 5.7% | 10.3% | 17.5% | 28.7% | 45.2% | 68.4% |
| 60 | 0.5% | 1.5% | 3.3% | 6.3% | 11.1% | 18.7% | 30.2% | 46.8% | 69.6% |
| 70 | 0.6% | 1.7% | 3.6% | 6.8% | 12.0% | 19.9% | 31.6% | 48.2% | 70.7% |
| 80 | 0.7% | 1.9% | 4.0% | 7.4% | 12.8% | 21.0% | 32.9% | 49.5% | 71.6% |
| 90 | 0.7% | 2.1% | 4.3% | 8.0% | 13.6% | 22.1% | 34.2% | 50.8% | 72.5% |
| 100 | 0.8% | 2.3% | 4.7% | 8.5% | 14.4% | 23.1% | 35.4% | 51.9% | 73.3% |

Tables 10A, 10B, and 10C have more detailed information about the peroxide to water ratio in the vapor phase at various temperatures and concentrations by recalculating the mole fraction data in the Table 9. Since hydrogen peroxide, H₂O₂, has one more oxygen than water, H₂O, the ratio of hydrogen peroxide to water based on the weight is larger than the ratio of hydrogen peroxide to water based on the mole.

Table 10A has the ratios of hydrogen peroxide to water in the vapor phase with 10%, 20% and 30% hydrogen peroxide solutions by mole under various temperatures.

**Table 10A**

| Ratio of peroxide to water in the vapor phase over hydrogen peroxide solutions | | | | | | |
|---|---|---|---|---|---|---|
| Temp. (°C) | 10% by mole or 17.3% by weight in solution | | 20% by mole or 32.1 % by weight in solution | | 30% by mole or 44.7% by weight in solution | |
| | Ratio of peroxide to water in vapor by mole | Ratio of peroxide to water in vapor by weight | Ratio of peroxide to water in vapor by mole | Ratio of peroxide to water in vapor by weight | Ratio of peroxide to water in vapor by mole | Ratio of peroxide to water in vapor by weight |
| 0 | 0.0020 | 0.0038 | 0.0060 | 0.0114 | 0.0152 | 0.0288 |
| 10 | 0.0030 | 0.0057 | 0.0081 | 0.0152 | 0.0183 | 0.0346 |
| 20 | 0.0030 | 0.0057 | 0.0091 | 0.0172 | 0.0204 | 0.0385 |
| 25 | 0.0030 | 0.0057 | 0.0101 | 0.0191 | 0.0225 | 0.0425 |
| 30 | 0.0030 | 0.0057 | 0.0101 | 0.0191 | 0.0235 | 0.0445 |
| 40 | 0.0040 | 0.0076 | 0.0121 | 0.0229 | 0.0267 | 0.0504 |
| 50 | 0.0050 | 0.0095 | 0.0142 | 0.0268 | 0.0309 | 0.0584 |
| 60 | 0.0050 | 0.0095 | 0.0152 | 0.0288 | 0.0341 | 0.0645 |
| 70 | 0.0060 | 0.0114 | 0.0173 | 0.0327 | 0.0373 | 0.0705 |
| 80 | 0.0070 | 0.0133 | 0.0194 | 0.0366 | 0.0417 | 0.0787 |
| 90 | 0.0070 | 0.0133 | 0.0215 | 0.0405 | 0.0449 | 0.0849 |
| 100 | 0.0081 | 0.0152 | 0.0235 | 0.0445 | 0.0493 | 0.0932 |

**Table 10B**

| Ratio of peroxide to water in the vapor phase over hydrogen peroxide solutions | | | | | | |
|---|---|---|---|---|---|---|
| Temp. (°C) | 40% by mole or 55.7% by weight in solution | | 50% by mole or 65.4% by weight in solution | | 60% by mole or 73.9% by weight in solution | |
| | Ratio of peroxide to water in vapor by mole | Ratio of peroxide to water in vapor by weight | Ratio of peroxide to water in vapor by mole | Ratio of peroxide to water in vapor by weight | Ratio of peroxide to water in vapor by mole | Ratio of peroxide to water in vapor by weight |
| 0 | 0.0320 | 0.0604 | 0.0638 | 0.1206 | 0.1261 | 0.2382 |
| 10 | 0.0384 | 0.0726 | 0.0753 | 0.1422 | 0.1468 | 0.2773 |
| 20 | 0.0428 | 0.0808 | 0.0834 | 0.1576 | 0.1601 | 0.3024 |
| 25 | 0.0460 | 0.0869 | 0.0881 | 0.1665 | 0.1682 | 0.3178 |
| 30 | 0.0482 | 0.0911 | 0.0929 | 0.1755 | 0.1779 | 0.3360 |
| 40 | 0.0549 | 0.1036 | 0.1038 | 0.1960 | 0.1947 | 0.3678 |
| 50 | 0.0604 | 0.1142 | 0.1148 | 0.2169 | 0.2121 | 0.4007 |
| 60 | 0.0672 | 0.1270 | 0.1249 | 0.2358 | 0.2300 | 0.4345 |
| 70 | 0.0730 | 0.1378 | 0.1364 | 0.2576 | 0.2484 | 0.4693 |
| 80 | 0.0799 | 0.1509 | 0.1468 | 0.2773 | 0.2658 | 0.5021 |
| 90 | 0.0870 | 0.1643 | 0.1574 | 0.2973 | 0.2837 | 0.5359 |
| 100 | 0.0929 | 0.1755 | 0.1682 | 0.3178 | 0.3004 | 0.5674 |

Table 10C has the hydrogen peroxide to water ratios in the vapor phase with 70%, 80% and 90% hydrogen peroxide solutions by mole.

**Table 10C**

| Ratio of peroxide to water in the vapor phase over hydrogen peroxide solutions | | | | | | |
|---|---|---|---|---|---|---|
| Temp. (°C) | 70% by mole or 81.5% by weight in solution | | 80% by mole or 88.3% by weight in solution | | 90% by mole or 94.4% by weight in solution | |
| | Ratio of peroxide to water in vapor by mole | Ratio of peroxide to water in vapor by weight | Ratio of peroxide to water in vapor by mole | Ratio of peroxide to water in vapor by weight | Ratio of peroxide to water in vapor by mole | Ratio of peroxide to water in vapor by weight |
| 0 | 0.2531 | 0.4781 | 0.5432 | 1.0261 | 1.5000 | 2.8333 |
| 10 | 0.2887 | 0.5452 | 0.6155 | 1.1626 | 1.6738 | 3.1616 |
| 20 | 0.3123 | 0.5900 | 0.6584 | 1.2436 | 1.7778 | 3.3580 |
| 25 | 0.3280 | 0.6196 | 0.6863 | 1.2964 | 1.8409 | 3.4773 |
| 30 | 0.3423 | 0.6465 | 0.7153 | 1.3511 | 1.9070 | 3.6021 |
| 40 | 0.3736 | 0.7057 | 0.7699 | 1.4543 | 2.0395 | 3.8524 |
| 50 | 0.4025 | 0.7603 | 0.8248 | 1.5580 | 2.1646 | 4.0886 |
| 60 | 0.4327 | 0.8173 | 0.8797 | 1.6617 | 2.2895 | 4.3246 |
| 70 | 0.4620 | 0.8726 | 0.9305 | 1.7576 | 2.4130 | 4.5578 |
| 80 | 0.4903 | 0.9261 | 0.9802 | 1.8515 | 2.5211 | 4.7621 |
| 90 | 0.5198 | 0.9818 | 1.0325 | 1.9503 | 2.6364 | 4.9798 |
| 100 | 0.5480 | 1.0351 | 1.0790 | 2.0381 | 2.7453 | 5.1856 |

By monitoring the concentration (i.e. the peroxide concentration or the ratio of hydrogen peroxide to water) during the sterilization cycle and controlling the timing to close the valve, it should be possible to achieve the long sought goal of parametric release. One could be assured that if the proper concentration was maintained for a sufficient period of time that a particular load of instruments placed within the chamber 30 and sterilized according to the present invention then the process would be sufficiently predictable so as to allow the load to be released for use without further checking with a biological indicator. Typically, such processes employ a biological indicator in the load, such as with a test load of microorganisms, which is then checked to ensure that sufficient sterilization has been achieved to kill all of the test microorganisms. With parametric release the time consuming process of biological verifications can be skipped.

As described previously, shipping hydrogen peroxide solution with more than 60% by weight is regulated and can be difficult and impractical. One of the goals for this concentrating process is to concentrate the hydrogen peroxide solution in the system from less than 60% by weight to greater than 60% by weight. Therefore, more concentrated hydrogen peroxide can be generated during the process for a more efficacious cycle.

The process may be further enhanced by admitting sufficient hydrogen peroxide into the system so as to force some of the vaporized solution to condense upon the instruments being sterilized within the system. As described above, the solution can be vaporized by admitting it into the system at any pressure above the vapor pressures of water and hydrogen peroxide in the solution and then vaporized by reducing the pressure, or by admitting the solution at a pressure substantially below its vapor pressure whereupon it will start to vaporize thus releasing gas and increasing the pressure. In the second scenario if the pressure is then further reduced by pumping down the system, the concentration of the hydrogen peroxide in the system can be increased. This is especially true if the peroxide partial pressure rises to a level at least above the equilibrium partial pressure of hydrogen peroxide thereby limiting further vaporization of hydrogen peroxide from solution and encouraging some of the hydrogen peroxide to condense upon objects such as instruments within the system. Some of the water vapor would likely also condense in such event. By controlling the pressure, excess water vapor would be exhausted from the system and then the condensed solution would re-vaporize. To the extent that such solution had condensed within diffusion restricted areas the re-vaporization therein would further increase the concentration in those areas to enhance the sterilization efficacy therein. The quantity of solution admitted will primarily determine the pressure rise to initiate such condensation. The process is described in more detail in JP-A-2000217893.

A typical cycle might comprise placing a load of instruments (not shown) within a CSR (Central Supply Room) wrapped tray within the chamber 30 (as shown in FIG 6) and then drawing a vacuum on the chamber 30 with the pump 32 down to below 1 torr or about 0.3 torr. An electromagnetic field applied to the chamber 30 at such time tends to drive any remaining water into the vapor or plasma phase so that the pump 32 can remove it. The pump 32 can be cycled or merely run continuously with the valve 34 controlling the vacuum process. Fresh dry air may be admitted to the chamber 30 raising the pressure back to atmosphere. Preferably the hydrogen peroxide solution, preferably a 59% hydrogen peroxide solution by weight, is admitted to the vaporizer 36 at atmospheric pressure and then the pump 32 exhausts the chamber 30 to a level at which the solution begins to vaporize. A sensor 120 for hydrogen peroxide vapor and sensor 122 (see FIG. 6) for water vapor in connection with an automated control system 124 can be employed to optimize the pressure conditions to enhance the initial vaporization and exhaust of water vapor. After the solution is sufficiently concentrated the temperature of the vaporizer 36 can be increased to rapidly vaporize the remaining solution. The valve 32 is closed to isolate the chamber 30 and the vaporized hydrogen peroxide solution is allowed to diffuse throughout the chamber to contact the instruments. Additional dry air or other gas can be admitted at this time to help push the sterilizing vapors into diffusion restricted areas, with the chamber 30 then further exhausted to resume a vacuum in the range of 2 to 10 torr. Additional admissions of air and vacuum can be employed, especially in connection with additional admission and concentration of hydrogen peroxide solutions. After the hydrogen peroxide vapors have diffused throughout the chamber for a sufficient time an electromagnetic field may be applied to drive the vapor into the plasma phase and effect further sterilization. When the field is removed the activated species formed from the hydrogen peroxide recombine as water and oxygen, leaving little residual hydrogen peroxide. The chamber can be raised to atmospheric pressure and the load removed.

It should be noted that the present invention is not limited to only those embodiments described in the Detailed Description. The invention is only limited by the scope of the following claims.

## Claims

1. An apparatus for sterilizing an article with a concentrated hydrogen peroxide vapor, the apparatus comprising:
a source of liquid germicide comprising hydrogen peroxide;
a chamber (90; 100) adapted to receive the article;
a first vaporizer (92; 101); and
a pump (94; 103),
wherein the first vaporizer (92; 101) and the pump (94; 103) are separately connected to a common port (91; 104) for fluid communication with the chamber (90; 100).

2. An apparatus according to claim 1 and further comprising a valve (96; 105) located to isolate the pump (94; 103) from the chamber (90; 100) and the vaporizer (92; 101).

3. An apparatus according to claim 1 and further comprising an inlet (98; 106) to bleed air into the apparatus, and wherein the inlet is located either in the chamber (90; 100) or between the vaporizer (92; 101) and the chamber.

4. An apparatus according to claim 3 wherein the inlet (98; 106) comprises a valve.

5. An apparatus according to claim 1 and further comprising a second vaporizer (102) in fluid communication with the chamber (90; 100) through the port (91; 104).

6. An apparatus according to claim 5 and further comprising a valve (107) between the first vaporizer (101) and the second vaporizer (102), to isolate the second vaporizer from the first vaporizer.

7. An apparatus according to claim 5 and further comprising an inlet (108) located between the first vaporizer and the second vaporizer, to bleed air into the apparatus.

8. A method of sterilizing an article using the apparatus of claim 1, said method comprising the steps of:
placing the article into said chamber (90; 100) containing an inner atmosphere;
introducing a solution comprising hydrogen peroxide and water into said vaporizer (92; 101), the solution having a ratio of hydrogen peroxide to water;
pumping a portion of the inner atmosphere out of the chamber (90; 100) by means of said pump (94; 103), thereby reducing pressure in the chamber and in the vaporizer (92; 101), and vaporizing water vapor out of the solution;
drawing the water vapor from the vaporizer (92; 101) to increase the ratio of hydrogen peroxide to water in the vaporizer;
terminating the pumping step without terminating the vaporizing steps;
vaporizing hydrogen peroxide vapor out of the solution and flowing the hydrogen peroxide vapor from the vaporizer (92; 101) into the chamber (90; 100); and
contacting the article with the hydrogen peroxide vapor for a time period sufficient to effect sterilization of the article.

9. A method according to claim 8 wherein the ratio of hydrogen peroxide to water in said solution, by weight, after the step of drawing water vapor from the vaporizer (92; 101) exceeds 3 to 1.

10. A method according to claim 9 wherein the ratio of hydrogen peroxide to water in said solution, by weight, is less than 3:2 before the step of drawing water vapor from the vaporizer (92; 101).

11. A method according to claim 9 wherein the ratio of hydrogen peroxide to water in said solution, by weight, after the step of drawing water vapor from the vaporizer (92; 101) exceeds 4 to 1

12. A method according to claim 8 wherein the step of drawing water vapor from the vaporizer (92; 101) comprises introducing said solution within a diffusion restricted environment in fluid communication with the chamber (90; 100) during the step of vaporizing the solution.

13. A method according to claim 12 wherein the diffusion restricted environment is more diffusion restricted during the step of drawing water vapor from the vaporizer (92; 101) than during a portion of the step of flowing the hydrogen peroxide vapor from the vaporizer into the chamber (90; 100).

14. A method according to claim 8 wherein at least a portion of the pumping step and the vaporizing step occur simultaneously.

15. A method according to claim 8 wherein at least a portion of the pumping step, the vaporizing step and the drawing step occur simultaneously.

16. A method according to claim 8 wherein the step of drawing water vapor from the vaporizer (92; 101) comprises the step of maintaining the solution at a pressure below the vapor pressure of the water in the solution and above the vapor pressure of the hydrogen peroxide in the solution.

17. A method according to claim 8 wherein the solution is vaporized by pumping a portion of the atmosphere out of the vaporizer (92; 101) to lower the pressure at a rate selected to control removal of the water and hydrogen peroxide from the solution so as to concentrate the hydrogen peroxide remaining in the vaporizer.

18. A method according to claim 8 wherein the temperature of the solution during the vaporizing step is held below the temperature of the atmosphere in the chamber (90; 100) whereby to increase the vapor pressure of the water in the solution relative to the hydrogen peroxide in the solution whereby to enhance vaporization of the water from the solution in preference to vaporizing the hydrogen peroxide from the solution.

19. A method according to claim 18 wherein the temperature of the atmosphere in the chamber (90; 100) is above room temperature and the temperature of the solution during the vaporizing step is at least 10°C below the temperature of the atmosphere in the chamber.

20. A method according to claim 18 wherein the vaporizer is thermally isolated from the chamber.

21. A method according to claim 8 and further comprising the steps of controlling the temperature and pressure of the solution during a least a first portion of the vaporizing step so as to vaporize water from the solution and concentrate hydrogen peroxide therein to form a concentrated solution and during the step of vaporizing hydrogen peroxide out of the solution raising the temperature of the concentrated solution and vaporizing the concentrated solution.

22. A method according to claim 8 wherein the step of contacting the article with the hydrogen peroxide vapor is limited to less than one hour and if the article were to have a straight round lumen having two open ends, a diameter of 1 mm and a length of 250mm with 10⁶ viable spores of *B. stearothermophilus* located within the lumen at a midpoint thereof, all of the spores would be killed.

23. A method according to claim 8 wherein the solution comprises peracetic acid.

24. A method according to claim 8 wherein the step of introducing the solution comprising hydrogen peroxide and water into vaporizer (92; 101) occurs at atmospheric pressure.

25. A method according to claim 8 wherein the step of introducing the solution comprising hydrogen peroxide and water into vaporizer (92; 101) occurs at the vapor pressure of said solution.

26. A method according to claim 8 wherein the step of introducing the solution comprising hydrogen peroxide and water into vaporizer (92; 101) occurs below the vapor pressure of said solution.

27. A method according to claim 8 and further comprising the step of bleeding air into said port (91; 104) from exterior of the chamber (90; 100).

28. A method according to claim 27 wherein said air is bled into the port (91; 104) from a location between the vaporizer (92; 101) and the chamber (90; 100).

29. A method according to claim 8 wherein the step of terminating the pumping step comprises closing fluid communication between the pump and the port (91; 104).

## Patentansprüche

1. Vorrichtung zum Sterilisieren eines Gegenstandes mit einem konzentrierten Wasserstoffperoxiddampf, wobei die Vorrichtung aufweist:
eine Quelle für flüssiges keimtötendes Mittel, das Wasserstoffperoxid aufweist;
eine Kammer (90; 100), die dazu ausgelegt ist, den Gegenstand aufzunehmen;
einen ersten Verdampfer (92; 101); und
eine Pumpe (94; 103),
wobei der erste Verdampfer (92; 101) und die Pumpe (94; 103) getrennt mit einem gemeinsamen Port (91; 104) für die fluidische Verbindung mit der Kammer (90; 100) verbunden sind.

2. Vorrichtung nach Anspruch 1 und weiter mit einem Ventil (96; 105), das so angeordnet ist, dass es die Pumpe (94; 103) von der Kammer (90; 100) und dem Verdampfer (92; 101) isoliert.

3. Vorrichtung nach Anspruch 1 und weiter mit einem Einlass (98; 106), um Luft in die Vorrichtung zu leiten, und bei der sich der Einlass entweder in der Kammer (90; 100) oder zwischen dem Verdampfer (92; 101) und der Kammer befindet.

4. Vorrichtung nach Anspruch 3, bei der der Einlass (98; 106) ein Ventil aufweist.

5. Vorrichtung nach Anspruch 1 und weiter mit einem zweiten Verdampfer (102) in fluidischer Verbindung mit der Kammer (90; 100) durch den Port (91; 104).

6. Vorrichtung nach Anspruch 5 und weiter mit einem Ventil (107) zwischen dem ersten Verdampfer (101) und dem zweiten Verdampfer (102), um den zweiten Verdampfer von dem ersten Verdampfer zu isolieren.

7. Vorrichtung nach Anspruch 5 und weiter mit einem Einlass (108), der sich zwischen dem ersten Verdampfer und dem zweiten Verdampfer befindet, um Luft in die Vorrichtung zu leiten.

8. Verfahren zum Sterilisieren eines Gegenstandes, bei dem die Vorrichtung nach Anspruch 1 verwendet wird, wobei das Verfahren die Schritte aufweist:
Bringen des Gegenstandes in die Kammer (90; 100), die eine innere Atmosphäre enthält;
Einführen einer Lösung, die Wasserstoffperoxid und Wasser aufweist, in den Verdampfer (92; 101), wobei die Lösung ein Verhältnis von Wasserstoffperoxid zu Wasser hat;
Abpumpen eines Teils der inneren Atmosphäre mit der Pumpe (94; 103) aus der Kammer (90; 100) heraus, so dass der Druck in der Kammer und in dem Verdampfer (92; 101) verringert wird, und Verdampfen von Wasserdampf aus der Lösung;
Abziehen des Wasserdampfes aus dem Verdampfer (92; 101), um das Verhältnis von Wasserstoffperoxid zu Wasser in dem Verdampfer zu erhöhen;
Beenden des Pumpschrittes ohne Beenden der Verdampfungsschritte;
Verdampfen von Wasserstoffperoxiddampf aus der Lösung und Einströmen des Wasserstoffperoxiddampfes aus dem Verdampfer (92; 101) in die Kammer (90; 100); und
Zusammenbringen des Gegenstandes mit dem Wasserstoffperoxiddampf über eine Zeitdauer, die ausreichend ist, das Sterilisieren des Gegenstandes zu bewirken.

9. Verfahren nach Anspruch 8, bei dem das Verhältnis von Wasserstoffperoxid zu Wasser in der Lösung, auf das Gewicht bezogen, nach dem Schritt des Abziehens von Wasserdampf aus dem Verdampfer (92; 101) 3 zu 1 übersteigt.

10. Verfahren nach Anspruch 9, bei dem das Verhältnis von Wasserstoffperoxid zu Wasser in der Lösung, auf das Gewicht bezogen, vor dem Schritt des Abziehens von Wasserdampf aus dem Verdampfer (92; 101) geringer als 3:2 ist.

11. Verfahren nach Anspruch 9, bei dem das Verhältnis von Wasserstoffperoxid zu Wasser in der Lösung, auf das Gewicht bezogen, nach dem Schritt des Abziehens von Wasserdampf aus dem Verdampfer (92; 101) 4 zu 1 übersteigt.

12. Verfahren nach Anspruch 8, bei dem der Schritt des Abziehens von Wasserdampf aus dem Verdampfer (92; 101) das Einführen der Lösung in eine diffusionsbeschränkte Umgebung in fluidischer Verbindung mit der Kammer (90; 100) während des Schrittes des Verdampfens der Lösung aufweist.

13. Verfahren nach Anspruch 12, bei dem die diffusionsbeschränkte Umgebung während des Schrittes des Abziehens von Wasserdampf aus dem Verdampfer (92; 101) stärker diffusionsbeschränkt ist als während eines Teiles des Schrittes des Einströmenlassens des Wasserstoffperoxiddampfes aus dem Verdampfer in die Kammer (90; 100).

14. Verfahren nach Anspruch 8, bei dem wenigstens ein Teil des Pumpschritts und der Verdampfungsschritt gleichzeitig geschehen.

15. Verfahren nach Anspruch 8, bei dem wenigstens ein Teil des Pumpschritts der Verdampfungsschritt und der Abziehschritt gleichzeitig geschehen.

16. Verfahren nach Anspruch 8, bei dem der Schritt des Abziehens von Wasserdampf aus dem Verdampfer (92; 101) den Schritt des Haltens der Lösung auf einem Druck unterhalb des Dampfdrucks des Wassers in der Lösung und oberhalb des Dampfdrucks des Wasserstoffperoxids in der Lösung aufweist.

17. Verfahren nach Anspruch 8, bei dem die Lösung verdampft wird, indem ein Teil der Atmosphäre aus dem Verdampfer (92; 101) abgepumpt wird, um den Druck mit einer Geschwindigkeit zu senken, die so gewählt ist, dass das Entfernen des Wassers und des Wasserstoffperoxides aus der Lösung derart gesteuert wird, dass das in dem Verdampfer verbleibende Wasserstoffperoxid konzentriert wird.

18. Verfahren nach Anspruch 8, bei dem die Temperatur der Lösung während des Verdampfungsschrittes unterhalb der Temperatur der Atmosphäre in der Kammer (90; 100) gehalten wird, wodurch der Dampfdruck des Wassers in der Lösung relativ zu dem Wasserstoffperoxid in der Lösung erhöht wird, so dass das Verdampfen des Wassers aus der Lösung gegenüber dem Verdampfen des Wasserstoffperoxids aus der Lösung vorrangig wird.

19. Verfahren nach Anspruch 18, bei dem die Temperatur der Atmosphäre in der Kammer (90; 100) oberhalb Zimmertemperatur liegt und die Temperatur der Lösung während des Verdampfungsschrittes wenigstens 10 °C unterhalb der Temperatur der Atmosphäre in der Kammer ist.

20. Verfahren nach Anspruch 18, bei dem der Verdampfer von der Kammer thermisch isoliert ist.

21. Verfahren nach Anspruch 8 und weiter mit den Schritten des Steuerns der Temperatur und des Druckes der Lösung während wenigstens einem ersten Teil des Verdampfungsschrittes, um so Wasser aus der Lösung zu verdampfen und Wasserstoffperoxid darin zu konzentrieren, um eine konzentrierte Lösung zu bilden, und, während des Schrittes des Verdampfens von Wasserstoffperoxid aus der Lösung, des Anhebens der Temperatur der konzentrierten Lösung und des Verdampfens der konzentrierten Lösung.

22. Verfahren nach Anspruch 8, bei dem der Schritt des Zusammenbringens des Gegenstandes mit dem Wasserstoffperoxiddampf auf weniger als eine Stunde beschränkt ist, und, falls der Gegenstand ein gerades rundes Lumen mit zwei offenen Enden, einen Durchmesser von 1 mm und eine Länge von 250 mm hat mit 10⁶ lebensfähigen Sporen des *B. stearothermophilus,* die sich innerhalb des Lumens an seinem Mittelpunkt befinden, alle Sporen getötet werden würden.

23. Verfahren nach Anspruch 8, bei dem die Lösung Peressigsäure aufweist.

24. Verfahren nach Anspruch 8, bei dem der Schritt des Einführens der Lösung, die Wasserstoffperoxid und Wasser aufweist, in den Verdampfer (92; 101) bei atmosphärischem Druck geschieht.

25. Verfahren nach Anspruch 8, bei dem der Schritt des Einführens der Lösung, die Wasserstoffperoxid und Wasser aufweist, in den Verdampfer (92; 101) bei dem Dampfdruck der Lösung geschieht.

26. Verfahren nach Anspruch 8, bei dem der Schritt des Einführens der Lösung, die Wasserstoffperoxid und Wasser aufweist, in den Verdampfer (92; 101) unterhalb des Dampfdruckes der Lösung geschieht.

27. Verfahren nach Anspruch 8 und weiter mit dem Schritt des Leitens von Luft in den Port (91; 104) von außerhalb der Kammer (90; 100).

28. Verfahren nach Anspruch 27, bei dem Luft in den Port (91; 104) von einem Ort zwischen dem Verdampfer (92; 101) und der Kammer (90; 100) geleitet wird.

29. Verfahren nach Anspruch 8, bei dem der Schritt des Beendens des Pumpschrittes das Schließen der fluidischen Verbindung zwischen der Pumpe und dem Port (91; 104) aufweist.

## Revendications

1. Appareil pour stériliser un objet avec une vapeur de peroxyde d'hydrogène concentrée, l'appareil comprenant :
■ une source de germicide liquide comprenant du peroxyde d'hydrogène ;
■ une chambre (90 ; 100) adaptée pour recevoir l'objet ;
■ un premier vaporisateur (92 ; 101) ; et
■ une pompe (94 ; 103) ;
dans lequel le premier vaporisateur (92 ; 101) et la pompe (94 ; 103) sont raccordés séparément à un orifice commun (91 ; 104) pour la communication de fluide avec la chambre (90 ; 100).

2. Appareil selon la revendication 1 et comprenant en outre une soupape (96 ; 105) positionnée pour isoler la pompe (94 ; 103) de la chambre (90 ; 100) et du vaporisateur (92 ; 101).

3. Appareil selon la revendication 1 et comprenant en outre une entrée (98 ; 106) pour purger l'air dans l'appareil, et dans lequel l'entrée est positionnée dans la chambre (90 ; 100) ou entre le vaporisateur (92 ; 101) et la chambre.

4. Appareil selon la revendication 3, dans lequel l'entrée (98 ; 106) comprend une soupape.

5. Appareil selon la revendication 1 et comprenant en outre un second vaporisateur (102) en communication de fluide avec la chambre (90 ; 100) par l'intermédiaire de l'orifice (91 ; 104).

6. Appareil selon la revendication 5 et comprenant en outre une soupape (107) entre le premier vaporisateur (101) et le second vaporisateur (102), pour isoler le second vaporisateur du premier vaporisateur.

7. Appareil selon la revendication 5 et comprenant en outre une entrée (108) située entre le premier vaporisateur et le second vaporisateur, pour purger l'air dans l'appareil.

8. Procédé pour stériliser un objet en utilisant l'appareil de la revendication 1, ledit procédé comprenant les étapes consistant à :
■ placer l'objet dans ladite chambre (90 ; 100) contenant une atmosphère interne ;
■ introduire une solution comprenant du peroxyde d'hydrogène et de l'eau dans ledit vaporisateur (92 ; 101), la solution ayant un rapport de peroxyde d'hydrogène sur l'eau ;
■ pomper une partie de l'atmosphère interne à l'extérieur de la chambre (90 ; 100) au moyen de ladite pompe (94 ; 103), réduisant ainsi la pression dans la chambre et dans le vaporisateur (92 ; 101), et vaporiser la vapeur d'eau sans la solution ;
■ aspirer la vapeur d'eau du vaporisateur (92 ; 101) pour augmenter le rapport du peroxyde d'hydrogène sur l'eau dans le vaporisateur ;
■ terminer l'étape de pompage sans achever les étapes de vaporisation ;
■ vaporiser la vapeur de peroxyde d'hydrogène sans la solution et laisser s'écouler la vapeur de peroxyde d'hydrogène du vaporisateur (92 ; 101) dans la chambre (90 ; 100) ; et
■ mettre en contact l'objet avec la vapeur de peroxyde d'hydrogène pendant une période de temps suffisante pour effectuer la stérilisation de l'article.

9. Procédé selon la revendication 8, dans lequel le rapport du peroxyde d'hydrogène sur l'eau dans ladite solution, en poids, après l'étape consistant à aspirer la vapeur d'eau du vaporisateur (92 ; 101) dépasse 3 sur 1.

10. Procédé selon la revendication 9, dans lequel le rapport de peroxyde d'hydrogène sur l'eau dans ladite solution, en poids, est inférieur à 3:2 avant l'étape consistant à aspirer la vapeur d'eau du vaporisateur (92 ; 101).

11. Procédé selon la revendication 9, dans lequel le rapport de peroxyde d'hydrogène sur l'eau dans ladite solution, en poids, après l'étape consistant à aspirer la vapeur d'eau du vaporisateur (92 ; 101) dépasse 4 sur 1.

12. Procédé selon la revendication 8, dans lequel l'étape consistant à aspirer la vapeur d'eau du vaporisateur (92 ; 101) comprend l'étape consistant à introduire ladite solution dans un environnement à diffusion limitée en communication de fluide avec la chambre (90 ; 100) pendant l'étape consistant à vaporiser la solution.

13. Procédé selon la revendication 12, dans lequel l'environnement à diffusion limitée a une diffusion plus limitée pendant l'étape consistant à aspirer la vapeur d'eau du vaporisateur (92 ; 101) que pendant une partie de l'étape consistant à laisser s'écouler la vapeur de peroxyde d'hydrogène du vaporisateur dans la chambre (90 ; 100).

14. Procédé selon la revendication 8, dans lequel au moins une partie de l'étape de pompage et une partie de l'étape de vaporisation se produisent simultanément.

15. Procédé selon la revendication 8, dans lequel au moins une partie de l'étape de pompage, une partie de l'étape de vaporisation et une partie de l'étape d'aspiration se produisent simultanément.

16. Procédé selon la revendication 8, dans lequel l'étape consistant à aspirer la vapeur d'eau du vaporisateur (92 ; 101) comprend l'étape consistant à maintenir la solution à une pression inférieure à la pression de vapeur de l'eau dans la solution et au-dessus de la pression de vapeur du peroxyde d'hydrogène dans la solution.

17. Procédé selon la revendication 8, dans lequel la solution est vaporisée en pompant une partie de l'atmosphère à l'extérieur du vaporisateur (92 ; 101) pour abaisser la pression à une vitesse sélectionnée pour contrôler le retrait de l'eau et du peroxyde d'hydrogène de la solution afin de concentrer le peroxyde d'hydrogène restant dans le vaporisateur.

18. Procédé selon la revendication 8, dans lequel la température de la solution pendant l'étape de vaporisation est maintenue au-dessous de la température de l'atmosphère dans la chambre (90 ; 100) afin d'augmenter la pression de vapeur de l'eau dans la solution par rapport au peroxyde d'hydrogène dans la solution, afin d'améliorer la vaporisation de l'eau de la solution par rapport à la vaporisation du peroxyde d'hydrogène de la solution.

19. Procédé selon la revendication 18, dans lequel la température de l'atmosphère dans la chambre (90 ; 100) est supérieure à la température ambiante et la température de la solution pendant l'étape de vaporisation est au moins 10°C au-dessous de la température de l'atmosphère dans la chambre.

20. Procédé selon la revendication 18, dans lequel le vaporisateur est thermiquement isolé de la chambre.

21. Procédé selon la revendication 8 et comprenant en outre les étapes consistant à contrôler la température et la pression de la température pendant au moins une première partie de l'étape de vaporisation afin de vaporiser l'eau de la solution et concentrer le peroxyde d'hydrogène pour former une solution concentrée et pendant l'étape de vaporisation du peroxyde d'hydrogène sans la solution, faire monter la température de la solution concentrée et vaporiser la solution concentrée.

22. Procédé selon la revendication 8, dans lequel l'étape consistant à mettre en contact l'objet avec la vapeur de peroxyde d'hydrogène est limitée à moins d'une heure et si l'objet doit avoir une lumière ronde droite ayant deux extrémités ouvertes, un diamètre de 1 mm et une longueur de 250 mm avec des spores de B. Stearothermophilus de 10⁶ viables situées à l'intérieur de la lumière au niveau de son point central, toutes les spores seraient tuées.

23. Procédé selon la revendication 8, dans lequel la solution comprend de l'acide péracétique.

24. Procédé selon la revendication 8, dans lequel l'étape consistant à introduire la solution comprenant du peroxyde d'hydrogène et de l'eau dans le vaporisateur (92 ; 101) se produit à la pression atmosphérique.

25. Procédé selon la revendication 8, dans lequel l'étape consistant à introduire la solution comprenant le peroxyde d'hydrogène et l'eau dans le vaporisateur (92 ; 101) se produit à la pression de vapeur de ladite solution.

26. Procédé selon la revendication 8, dans lequel l'étape consistant à introduire la solution comprenant le peroxyde d'hydrogène et l'eau dans le vaporisateur (92 ; 101) se produit au-dessous de la pression de vapeur de ladite solution.

27. Procédé selon la revendication 8 et comprenant en outre l'étape consistant à purger l'air dans ledit orifice (91 ; 104) de l'extérieur de la chambre (90 ; 100).

28. Procédé selon la revendication 27, dans lequel ledit air est purgé dans l'orifice (91 ; 104) d'un emplacement situé entre le vaporisateur (92 ; 101) et la chambre (90 ; 100).

29. Procédé selon la revendication 8, dans lequel l'étape consistant à achever l'étape de pompage comprend l'étape consistant à fermer la communication de fluide entre la pompe et l'orifice (91 ; 104).
